Europäisches Patentamt

European Patent Office

Office européen des brevets

(11) Publication number: **0 436 318 A1**

## EUROPEAN PATENT APPLICATION

(21) Application number: 90313411.2

(51) Int. Cl.5: **A61B 5/00**

(22) Date of filing: **11.12.90**

(30) Priority: **29.12.89 US 458967**

(43) Date of publication of application:
**10.07.91 Bulletin 91/28**

(84) Designated Contracting States:
**AT BE CH DE DK ES FR GB GR IT LI LU NL SE**

(71) Applicant: **SENTINEL MONITORING, INC.**
**6530 Corporate Drive**
**Indianapolis, Indiana 46278(US)**

(72) Inventor: **Willett, Ronald P.**
**1821 Arden Drive**
**Bloomington, Indiana 47401(US)**

(74) Representative: **Thomson, Roger Bruce**
**W P THOMPSON & CO, Eastcheap House,**
**Central Approach**
**Letchworth Hertfordshire SG6 3DS(GB)**

(54) Sensor applicator.

(57) A sensor applicator (12) for measuring a phys-
iological property of a patient includes a housing
(14) for holding a sensor (16), an elastic strap (18)
for securing the sensor applicator against the patient,
and a clasp (32) for adjusting the elastic strap with
respect to the size of the patient and the amount of
force that is placed on said sensor against the pa-
tient.

FIG. 1

EP 0 436 318 A1

## SENSOR APPLICATOR

The present invention relates to a sensor applicator for use in securely holding a sensor on a patient.

The non-invasive measuring of certain physiological properties has long been practised by physicians in the prior art. Such techniques include using sensors which transmit one or more wavelengths of radiation into perfused tissue and detecting light passing through or being reflected from the tissue, and using the signal obtained to determine various physiological quantities, such as oxyhaemoglobin saturation. A common problem for these sensors is the amount of pressure applied by the sensor against the tissue. It is important that the sensor should press firmly against the tissue in order efficiently to use the radiation being transmitted and minimise radiation leakage problems with the sensor. However, too much pressure will cause blood to leave the tissue making it more difficult to obtain accurate measurement. Thus, a delicate balance is needed in order to optimise performance of the sensor. Additionally, patient comfort during prolonged use of the device becomes important. Typically, in the prior art, adhesive tapes have been used to apply sensors to the body. However, this type of application is limited to areas of patients which are dry, clean, and generally flat, for example foreheads. Over time these adhesive tapes tend to loosen, which affects the ability of the sensors to operate properly. Additionally, use of adhesive tape is difficult for prolonged use, or on individuals who are diaphoretic or sweaty. This generally causes the sensor to loosen from the skin, and thus may provide inaccurate measurements.

It is an object of the invention to provide an improved sensor applicator which minimises or eliminates many of the problems of the prior art.

A sensor applicator made in accordance with the present invention provides the appropriate pressure of the sensor against the patient such that the sensor is firmly and securely held in position, but does not provide too much pressure which will either cause blood to leave the tissue or become discomforting to the patient during prolonged use. Additionally, the applicator, according to the present invention, provides means for securely holding the sensor against the patient even when the patient is diaphoretic or sweaty.

In accordance with one aspect of the invention there is provided a sensor assembly for measuring a physiological property of a patient comprising:

a) a sensor having means for measuring a physiological property of a patient;

b) sensor housing means for holding said sensor;

c) elastic means for securing said sensor housing means to said patient, said elastic means being capable of being wrapped about a portion of the patient; and

d) adjustable means for adjusting the size of said elastic means with respect to said patient, and the amount of force that is placed on said sensor against said patient.

In accordance with another aspect of the invention there is provided a sensor applicator for holding a sensor for measuring a physiological property of a patient, comprising:

a) a sensor housing for holding a sensor for measuring a physiological property of a patient;

b) elastic means for securing said sensor holding means to said patient, said elastic means being capable of being wrapped about a portion of the patient; and

c) adjustable means for adjusting the size of said elastic means with respect to said patient, and the amount of force that is placed on said sensor against said patient.

A preferred embodiment of the invention will now be described by way of example and with reference to the accompanying drawings, in which:

Figure 1 is a side elevational view of a sensor applicator in accordance with the present invention as mounted against the forehead of a patient;

Figure 2 is a partial perspective view of the sensor applicator of Figure 1.

Figure 3 is a partial bottom plan view of the sensor applicator illustrated in Figure 1;

Figure 4 is a side elevational view of Figure 3;

Figure 5 is a top plan view of the sensor applicator of Figure 1;

Figure 6 is a top view of the sensor applicator taken along line 6-6 of Figure 1;

Figure 7 is an enlarged view of the portion of the applicator indicated by zone 7-7 of Figure 6;

Figure 8 is an enlarged partial view of the portion of the applicator of Figure 6 indicated by zone 8-8; and

Figure 9 is an enlarged plan view of the buckle used to secure the strap as illustrated in Figure 8.

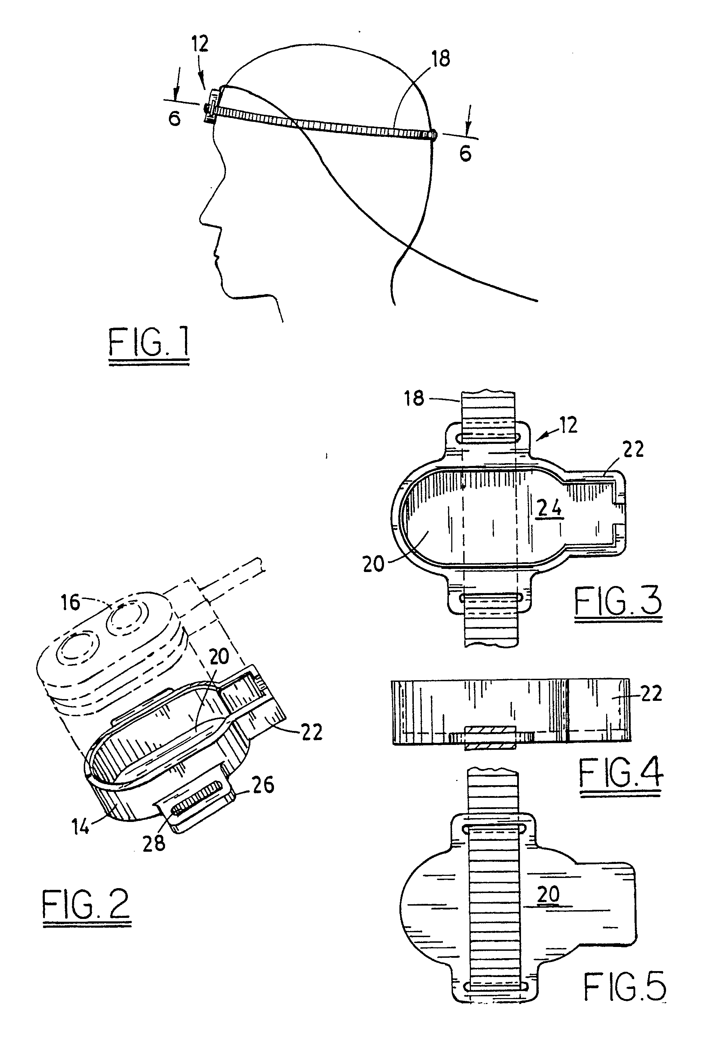

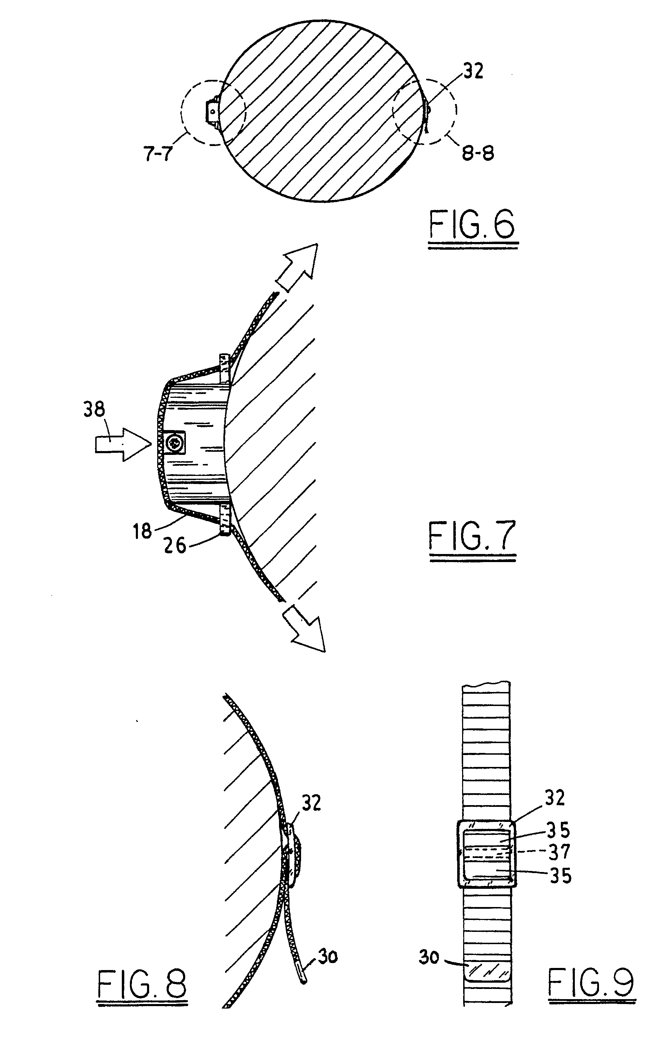

Referring to Figures 1 to 9, there is illustrated a sensor applicator 12 made in accordance with the present invention. The sensor applicator 12 includes a housing 14 capable of receiving and holding a sensor 16 (shown in phantom lines in Figure 2) for measuring a physiological property of a patient, and a strap 18 for securing the sensor assembly to a patient. In the particular embodiment illustrated, the sensor 16 is an oximeter sensor designed to measure oxyhaemoglobin saturation such as described in our co-pending US patent application Serial No. 07/452700. It is to be understood that various other sensors may be used as desired for measuring the desired physiological property of the patient.

The housing 14 includes a base 20 having an upstanding outer peripheral wall 22. Base 20 and peripheral wall 22 form a receiving chamber 24 having an inner configuration which corresponds substantially to the outer configuration of the sensor 16 to be placed therein. It is, of course, understood that the configuration of base 20 and peripheral wall 22 may be designed to correspond to the appropriate outer configuration of any desired sensor to be used. The peripheral wall 22 is designed such that the sensor 16 will extend above the top of peripheral wall 22 so that the sensor can properly engage the patient. Disposed on either side of the peripheral wall 22 is a pair of integrally formed projecting members 26 each having an elongated opening 28, sized such that a strap 18 may easily pass therethrough. In the particular embodiment illustrated, the projecting members 26 are placed adjacent to base 20. In the particular embodiment illustrated, housing 14 is moulded from an appropriate plastics material.

The strap 18 has a pair of outer ends 30 which are capable of being joined together by an adjustable clasp 32 which allows the ends 30 of strap 18 to pass therethrough such that the ends 30 of strap 18 will maintain their position during use. In the particular embodiment illustrated, the clasp 32 comprises a generally flat member having a pair of adjacent openings 35 separated by a bar 37. The ends 30 pass by each other and through both openings 35. The amount by which ends 30 are passed through openings 35 will determine the size of the closed loop formed by strap 18 for receiving a portion of the patient and the amount of force applied against the patient. It is, of course, understood that any desired clasp may be used as desired as long as it joins the ends 30 together in an adjustable manner. The strap extends through the openings 28 such that a portion of the strap 18 extends behind the base 20 as illustrated in Figure 7. Preferably, as illustrated, the strap 18 is disposed about the central area of the base 20 so as to provide a substantially perpendicular force against the patient as indicated by arrow 38. The strap 18, in the particular embodiment illustrated, is made out of an appropriate elastic material as is commonly found in the prior art which is capable of elongating in a manner similar to a rubber band. In the particular embodiment illustrated, strap 18 is made of about 23% rubber and 77% polyester.

The manner in which the device may be used on a patient will now be described. Initially, the sensor applicator 12 is selected to be used with an appropriate sensor 16 and the sensor 16 is simply placed into the receiving chamber 24. The sensor assembly 10 is then placed against the patient in an appropriate manner. For example, as illustrated in the preferred embodiment, the sensor applicator 12 is placed against the forehead of a patient. However, sensor applicator 12 may be placed against any desired portion of the patient, for example the wrist, ankle, or digit. Thereafter, the ends 30 of the strap 18 are placed about the portion of the body to be secured, the ends 30 being fed through the adjustable clasp 32, or if the ends 30 have already been placed therethrough, the clasp 32 is moved to accommodate the size of the patient. The strap 18 and clasp 32 form a closed loop around the patient which is capable of compensating for the size and shape of the portion of the patient's body about which it is wrapped. The elastomeric strap 18 further provides means for applying the appropriate force on the sensor 16 against the patient. Preferably as illustrated, elastomeric strap 18 provides a force against the central area of the base 20 of housing 14 so as to cause the sensor assembly to be directed towards the patient. Thus, if the patient is wet or is diaphoretic, the tension provided by the strap 18 on the housing 14 will keep the sensor 16 securely and firmly in position against the patient. The strap 18 and clasp 32 not only provide means for adjusting for the size of the patient, but also provide means for adjusting the amount of force that is placed against the patient as required. Also, the amount of pressure being applied can be adjusted at any time during use to accommodate the patient's needs. Additionally, the applicator design provides means for easily moving the sensor to the optimal sensor application site on the patient. The sensor applicator 12 and sensor 16 provides an assembly for measuring a physiological property of a patient.

The sensor applicator may thus be used to maintain a sensor against a portion of a patient in a manner which is not only comfortable for long protracted use, but also minimises problems of diaphoretic or sweaty patients, thus allowing the sensor to maintain its proper operating function.

It is, of course, to be understood that various changes and modifications can be made without departing from the scope of the present invention. For example, the housing 14 may be made out of any desired material and the strap may be made out of any other extendable material.

## Claims

1. A sensor assembly for measuring a physiological property of a patient comprising:

a) a sensor (16) having means for measuring a physiological property of a patient;

b) sensor housing means (14) for holding said sensor (16);

c) elastic means (18) for securing said sensor housing means (14) to said patient, said elastic means being capable of being wrapped about a portion of the patient; and

d) adjustable means (32) for adjusting the size of said elastic means with respect to said patient, and the amount of force that is placed on said sensor (16) against said patient.

2. A sensor assembly according to claim 1, characterised in that said elastic means (18) is secured to said sensor housing means such that it provides a force against the back of said housing so as to cause said housing to be pressed in a direction towards said patient.

3. A sensor assembly according to claim 2, characterised in that said elastic means (18) is placed substantially centrally on said housing means (14).

4. A sensor assembly according to any preceding claim, characterised in that said elastic means comprises an elastic strap (18) having two free ends (30), said adjustable means (32) being secured to said free ends.

5. A sensor assembly according to any preceding claim, characterised in that said sensor housing means comprises a recess portion (24) having an internal configuration which corresponds to the external configuration of said sensor (16).

6. A sensor assembly according to any preceding claim, characterised in that said housing means includes a pair of projections (26) for allowing said elastic means (18) to pass therethrough and behind said housing means.

7. A sensor assembly according to any preceding claim, characterised in that said elastic means comprises a strap (18) made of about 23% rubber and about 77% polyester.

8. A sensor assembly according to any preceding claim, characterised in that said adjustable means comprises a clasp (32) placed at the free ends of said elastic means (18) such that they may be assembled in a manner so as to form a closed loop around a portion of said patient.

9. A sensor assembly according to any preceding claim, characterised in that said sensor (16) is designed to be used as an oximeter sensor.

10. A sensor applicator for holding a sensor for measuring a physiological property of a patient, comprising:

a) a sensor housing (14) for holding a sensor (16) for measuring a physiological property of a patient;

b) elastic means (18) for securing said sensor holding means to said patient, said elastic means being capable of being wrapped about a portion of the patient; and

c) adjustable means (32) for adjusting the size of said elastic means with respect to said patient, and the amount of force that is placed on said sensor against said patient.

11. A sensor applicator according to claim 10, characterised in that said elastic means (18) is secured to said sensor housing (14) such that it provides a force against the back of said sensor holding means so as to cause said sensor holding means to be pressed in a direction towards said patient.

12. A sensor applicator according to claim 10 or 11, characterised in that said elastic means (18) is placed substantially centrally on said sensor housing (14).

13. A sensor applicator according to claim 10, 11 or 12, characterised in that said elastic means comprises an elastic strap (18) having two free ends (30), said adjustable means (32) being secured to said free ends.

14. A sensor applicator according to any of claims 10 to 13, characterised in that said sensor housing comprises a recess portion (24) having an internal configuration which corresponds to the external configuration of the sensor it is to receive.

15. A sensor applicator according to any of claims 10 to 14, characterised in that said housing includes a pair of projections (26) which allow said elastic means to pass therethrough and behind said housing.

16. A sensor applicator according to any of claims 10 to 15, characterised in that said elastic means (18) is made of a material having about 23% rubber and 77% polyester.

17. A sensor applicator according to any of claims 10 to 16, characterised in that said adjustable means comprises a buckle (32) placed at the free ends of said elastic means such that said elastic means is assembled in a manner so as to form a closed loop around a portion of a patient.

18. A sensor applicator according to any of claims 10 to 17, characterised in that said sensor is designed to be used as an oximeter sensor.

FIG. 1

FIG.2

FIG.3

FIG.4

FIG.5

FIG.6

FIG.7

FIG.8

FIG.9

European
Patent Office

EUROPEAN SEARCH
REPORT

Application Number

EP 90 31 3411

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-2 630 486 (DORNIER SYSTEM G.M.B.H.) <br> * page 5, line 26 - page 6, line 23; figure 1 * | 1-4,8-13, 17,18 | A 61 B <br> 5/00 |
| X | US-A-4 380 240 (F.F. JÖBSIS ET AL.) <br> * column 6, lines 3 - 35; figures * | 1-5,9-14, 18 | |
| X | EP-A-0 329 196 (NELLCOR, INC.) <br> * page 4, lines 11 - 40; figures 1-3 * | 1-5,9-14, 18 | |
| X | US-A-4 640 295 (P.O. ISAACSON) <br> * column 2, line 44 - column 3, line 57 * * column 4, lines 40 - 54; figures * | 1-3,5,6, 10-12,14, 15 | |
| Y,A | US-A-3 831 586 (P.H. PETIT) <br> * column 2, line 59 - column 3, line 67 * * figures * | 1-4, 10-13,5, 14 | |
| Y,A | US-A-3 820 529 (R.L. GAUSE ET AL.) <br> * column 2, lines 3 - 48; figures * | 1-4, 10-13,8, 17 | |

| | TECHNICAL FIELDS SEARCHED (Int. Cl.5) |
|---|---|
| | A 61 B |

The present search report has been drawn up for all claims

| Place of search | Date of completion of search | Examiner |
|---|---|---|
| The Hague | 09 April 91 | RIEB K.D. |